# EUROPEAN PATENT APPLICATION

(11) **EP 4 851 282 A1**
(43) Date of publication of application: **09.12.2026**
(21) Application number: 25749171.2
(22) Date of filing: 31.01.2025
(51) Int. Cl.: C12N 9/78, C12N 1/15, C12N 1/19, C12N 1/21, C12N 5/10, C12N 15/55, C12N 15/63, C12P 21/00

(54) **MODIFIED PROTEIN GLUTAMINASE**

(30) Priority: 01.02.2024 JP 2024014090; 07.05.2024 JP 2024075344
(71) Applicant: Amano Enzyme Inc., Nagoya-shi Aichi 460-8630 (JP)
(72) Inventor: YOSHIDA, Kazunori, Kakamigahara-shi, Gifu 509-0109 (JP); SAKAI, Kiyota, Kakamigahara-shi, Gifu 509-0109 (JP); SUGIURA, Mahiru, Kakamigahara-shi, Gifu 509-0109 (JP)
(74) Representative: Vossius & Partner Patentanwälte Rechtsanwälte mbB
(86) International application number: PCT/JP2025/003316
(87) International publication number: WO 2025/164794

(57) **Abstract**

The purpose of the present invention is to provide a protein glutaminase that has an optimum temperature in a temperature region higher than the optimum temperature of a protein glutaminase derived from C. proteolyticum. A protein glutaminase comprising a polypeptide that comprises an amino acid sequence in which, in the amino acid sequence indicated by SEQ ID NO: 1, at least one substitution has been introduced from among a substitution of the second amino acid residue to a valine residue, a substitution of the 113th amino acid residue to an alanine residue, a substitution of the 154th amino acid residue to an aspartic acid residue, and a substitution of the 158th amino acid residue to a histidine residue has an optimum temperature in a temperature region higher than that of a polypeptide comprising the amino acid sequence indicated by SEQ ID NO: 1.

## Description

### Technical Field

The present invention relates to a modified protein glutaminase. More specifically, the present invention relates to a protein glutaminase modified to increase an optimum temperature.

### Background Art

A protein glutaminase is an enzyme that acts on protein, which is a high-molecular-weight substance, and catalyzes a reaction in which an amide group-containing side chain is decomposed (i.e., deamidated) without cleavage of peptide bonds and without crosslinking of the protein. Since the protein glutaminase deamidates glutamine residues in protein to generate negatively charged carboxyl groups, the protein glutaminase brings about various characteristic changes to the protein. For example, an increase in hydration force and a rise in electrostatic repulsion due to a decrease in isoelectric point of protein lead to a decrease in interaction between proteins (i.e., a decrease in association property), and thus enhance the solubility and water dispersibility of the protein. The exposure of inner hydrophobic regions due to a change in higher order structure of protein imparts interfacial activity to the protein, and thus improves the emulsifying power, emulsion stability, foaming property, and foam stability of the protein. Since the protein glutaminase can significantly change the properties of protein as described above, the protein glutaminase has dramatically expanded the applications of protein. Therefore, the protein glutaminase is very useful, and has attracted much attention in the art.

The first discovered protein glutaminase is a protein glutaminase found from Chryseobacterium proteolyticum in 2000 (NPL 1). Since then, a protein glutaminase derived from C. proteolyticum has been industrially used as the only active ingredient of a protein glutaminase enzyme preparation.

### Citation List

### Non Patent Literature

NPL 1: A novel protein-deamidating enzyme from Chryseobacterium proteolyticum sp. nov., a newly isolated bacterium from soil. Applied and Environmental Microbiology 2000; 66(8): 3337-43

### Summary of Invention

### Technical Problem

The protein glutaminase derived from C. proteolyticum is highly useful in that the optimum temperature is as high as 60°C, but the reactivity is greatly reduced at a temperature higher than the optimum temperature. Therefore, in the present situation, when the protein glutaminase derived from C. proteolyticum is used, temperature conditions should be strictly restricted so as not to exceed 60°C in consideration of the intrinsic thermal properties of the enzyme.

On the other hand, in view of the possibility of further expansion of the applications expected from high usefulness of protein glutaminase, and the like, it is desirable to create a protein glutaminase having an optimum temperature in a temperature region higher than the optimum temperature of protein glutaminase derived from C. proteolyticum.

Therefore, an object of the present invention is to provide a protein glutaminase having an optimum temperature in a temperature region higher than an optimum temperature of a protein glutaminase derived from C. proteolyticum.

### Solution to Problem

As a result of intensive studies, the present inventors have found a new mutation that improves the optimum temperature in protein glutaminase derived from C. proteolyticum. The present invention has been completed based on this finding.

That is, the present invention provides inventions of the following aspects.

Item 1. A modified protein glutaminase including a polypeptide shown in any one of (I) to (III) below:
(I) a polypeptide consisting of an amino acid sequence in which, in the amino acid sequence set forth in SEQ ID NO: 1, at least one substitution has been introduced from among a substitution of the amino acid residue at position 2 to a valine residue, a substitution of the amino acid residue at position 113 to an alanine residue, a substitution of the amino acid residue at position 154 to an aspartic acid residue, and a substitution of the amino acid residue at position 158 to a histidine residue;
(II) a polypeptide in which one or several amino acid residues other than the substituted amino acid residues are substituted, added, inserted, or deleted in the amino acid sequence in which the substitution has been introduced, and an optimum temperature is higher than an optimum temperature of a polypeptide consisting of the amino acid sequence set forth in SEQ ID NO: 1, and
(III) a polypeptide in which a sequence identity of regions excluding the substituted amino acid residues is 70% or more in the amino acid sequence in which the substitution has been introduced, and an optimum temperature is higher than an optimum temperature of a polypeptide consisting of the amino acid sequence set forth in SEQ ID NO: 1.

Item 2. A DNA encoding the modified protein glutaminase described in Item 1.
Item 3. An expression cassette or recombinant vector containing the DNA described in Item 2.
Item 4. A transformant obtained by transforming a host using the expression cassette or recombinant vector described in Item 3.
Item 5. A method for producing a modified protein glutaminase, the method including a step of culturing the transformant described in Item 4.
Item 6. An enzyme preparation containing the modified protein glutaminase described in Item 1.
Item 7. A modifier for a protein material, including the modified protein glutaminase described in Item 1.
Item 8. A method for producing a modified protein material, the production method including an enzyme treatment step of allowing the modified protein glutaminase described in Item 1 to act on a protein material.
Item 9. The production method described in Item 8, in which the protein material is a plant protein material.
Item 10. The production method described in Item 8 or 9, in which the protein material is provided in the form of a plant protein-containing liquid composition prepared using cereal crops, and
   in the enzyme treatment step, the plant protein-containing liquid composition is simultaneously treated with a modified protein glutaminase and an amylase.

### Advantageous Effects of Invention

According to the present invention, there is provided a protein glutaminase having an optimum temperature in a temperature region higher than an optimum temperature of a protein glutaminase derived from C. proteolyticum.

### Brief Description of Drawings

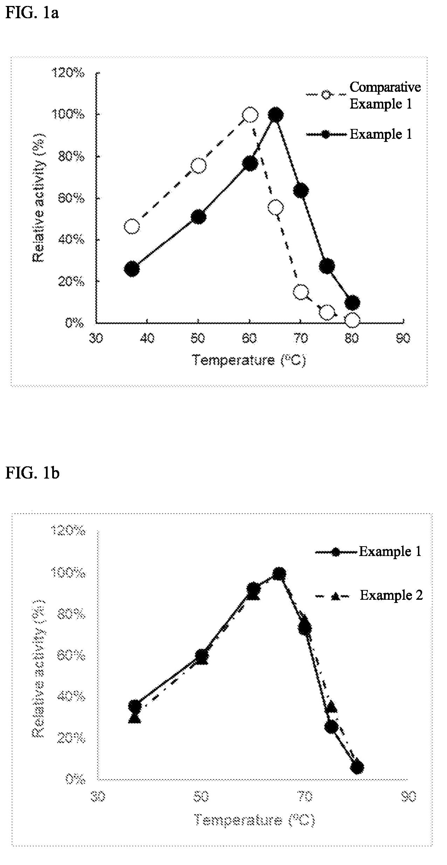

[Fig. 1a] Fig. 1a shows a comparison of the temperature dependence of the specific activity of protein glutaminase derived from C. proteolyticum (Comparative Example 1, dashed line) and the specific activity of a modified protein glutaminase of the present invention (Example 1, solid line) when Z-Gln-Gly is used as a substrate.
[Fig. 1b] Fig. 1b shows a comparison of the temperature dependence of the specific activity of a modified protein glutaminase of the present invention (Example 1, solid line) and the specific activity of a modified protein glutaminase of the present invention (Example 2, dashed-dotted line) when Z-Gln-Gly is used as a substrate.

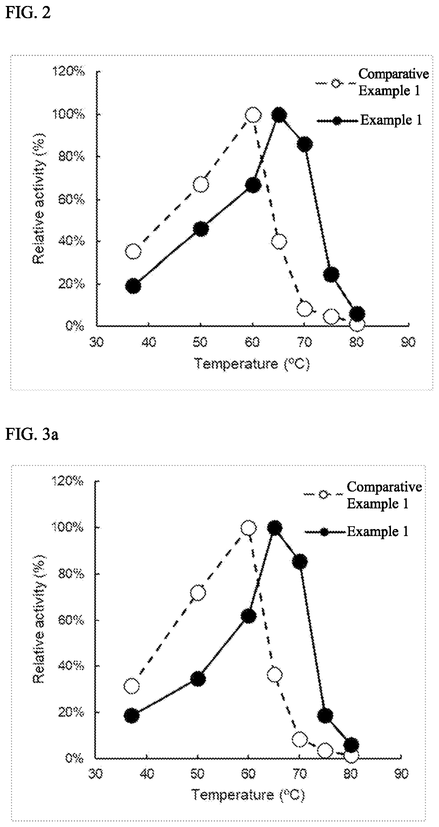

[Fig. 2] Fig. 2 shows a comparison of the temperature dependence of the specific activity of protein glutaminase derived from C. proteolyticum (Comparative Example 1, dashed line) and the specific activity of a modified protein glutaminase of the present invention (Example 1, solid line) when soybean protein is used as a substrate.
[Fig. 3a] Fig. 3a shows a comparison of the temperature dependence of the specific activity of protein glutaminase derived from C. proteolyticum (Comparative Example 1, dashed line) and the specific activity of a modified protein glutaminase of the present invention (Example 1, solid line) when pea protein is used as a substrate.

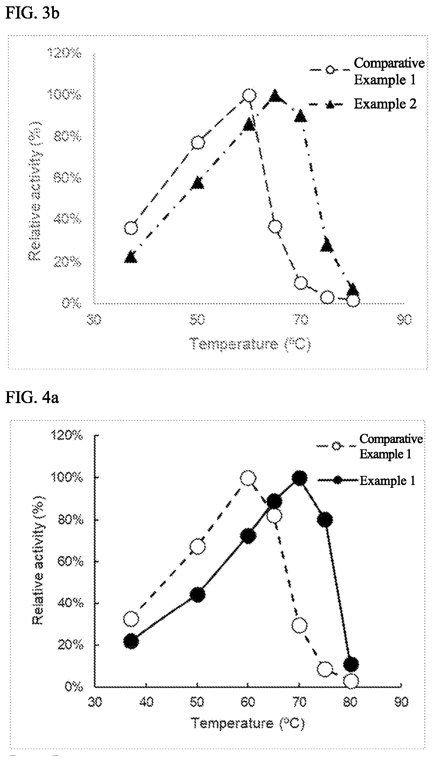

[Fig. 3b] Fig. 3b shows a comparison of the temperature dependence of the specific activity of protein glutaminase derived from C. proteolyticum (Comparative Example 1, dashed line) and the specific activity of a modified protein glutaminase of the present invention (Example 2, dashed-dotted line) when pea protein is used as a substrate.
[Fig. 4a] Fig. 4a shows a comparison of the temperature dependence of the specific activity of protein glutaminase derived from C. proteolyticum (Comparative Example 1, dashed line) and the specific activity of a modified protein glutaminase of the present invention (Example 1, solid line) when milk casein is used as a substrate.

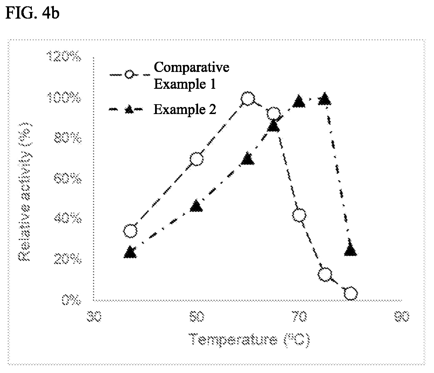

[Fig. 4b] Fig. 4b shows a comparison of the temperature dependence of the specific activity of protein glutaminase derived from C. proteolyticum (Comparative Example 1, dashed line) and the specific activity of a modified protein glutaminase of the present invention (Example 2, dashed-dotted line) when milk casein is used as a substrate.

### Description of Embodiments

Hereinafter, the present invention will be described in detail. Note that 20 amino acid residues in amino acid sequences may be abbreviated as one-letter codes. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the present specification, the amino acid sequence to be displayed is N-terminal at the left end and C-terminal at the right end.

In the present specification, the term "non-polar amino acid" includes glycine, alanine, valine, leucine, isoleucine, proline, methionine, phenylalanine, and tryptophan. The term "non-charged amino acid" includes serine, threonine, cysteine, tyrosine, asparagine, and glutamine. The term "acidic amino acid" includes aspartic acid and glutamic acid. The term "basic amino acid" includes lysine, arginine, and histidine.

In the present specification, the term "substitution" includes not only a case where a substitution of an amino acid residue is artificially introduced, but also a case where a substitution of an amino acid residue is naturally introduced, that is, a case where amino acid residues are originally different. In the present specification, the substitution of an amino acid residue may be an artificial substitution or a natural substitution, but an artificial substitution is preferred.

### 1. Modified protein glutaminase

The modified protein glutaminase of the present invention includes a polypeptide shown in any one of the following (I) to (III):
(I) a polypeptide consisting of an amino acid sequence in which, in the amino acid sequence set forth in SEQ ID NO: 1, at least one substitution has been introduced from among a substitution of the amino acid residue at position 2 to a valine residue, a substitution of the amino acid residue at position 113 to an alanine residue, a substitution of the amino acid residue at position 154 to an aspartic acid residue, and a substitution of the amino acid residue at position 158 to a histidine residue;
(II) a polypeptide in which one or several amino acid residues other than the substituted amino acid residues are substituted, added, inserted, or deleted in the amino acid sequence in which the substitution has been introduced, and an optimum temperature is higher than an optimum temperature of a polypeptide consisting of the amino acid sequence set forth in SEQ ID NO: 1; and
(III) a polypeptide in which a sequence identity of regions excluding the substituted amino acid residues is 70% or more in the amino acid sequence in which the substitution has been introduced, and an optimum temperature is higher than an optimum temperature of a polypeptide consisting of the amino acid sequence set forth in SEQ ID NO: 1.

The amino acid sequence set forth in SEQ ID NO: 1 is a mature sequence of protein glutaminase derived from Chryseobacterium proteolyticum.

In the polypeptide of the above (I), at least one substitution (hereinafter, also referred to as "predetermined substitution") may be introduced from among four substitutions consisting of a substitution of the amino acid residue at position 2 to a valine residue, a substitution of the amino acid residue at position 113 to an alanine residue, a substitution of the amino acid residue at position 154 to an aspartic acid residue, and a substitution of the amino acid residue at position 158 to a histidine residue. A preferred embodiment of the polypeptide of the above (I) is composed of the amino acid sequence set forth in SEQ ID NO: 2 into which all four substitutions have been introduced.

In the polypeptide of the above (II), amino acid modifications introduced may include any one of the modifications including substitution, addition, insertion, and deletion alone (for example, substitution alone) or include two or more of the modifications (for example, substitution and insertion). The number of amino acid differences at an arbitrary difference site in the polypeptide of the above (II) may be 1 or several, and is, for example, 1 to 55, 1 to 37, or 1 to 27, preferably 1 to 18, more preferably 1 to 10, further preferably 1 to 8, 1 to 7, 1 to 6, 1 to 5, or 1 to 4, still more preferably 1 to 3, and particularly preferably 1 or 2, or 1.

In the polypeptide of the above (III), the sequence identity to the amino acid sequence set forth in SEQ ID NO: 1 may be 70% or more, and is preferably 80% or more, or 85% or more, more preferably 90% or more or 93% or more, further preferably 95% or more, even more preferably 98% or more, still more preferably 98.5% or more or 99% or more, and particularly preferably 99.3% or more or 99.5% or more.

Herein, in the polypeptide of the above (III), the sequence identity to the amino acid sequence set forth in SEQ ID NO: 1 refers to a sequence identity calculated by comparison with the amino acid sequence set forth in SEQ ID NO: 1. The "sequence identity" refers to a value of amino acid sequence identity obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. Parameter settings may be as follows: Gap insertion Cost value: 11 and Gap extension Cost value: 1.

In the polypeptides of the above (II) and (III), amino acid residues corresponding to position 42 (cysteine residue), position 83 (histidine residue), and position 103 (aspartic acid residue) in the amino acid sequence set forth in SEQ ID NO: 1 are considered to be active catalyst residues, and thus it is desirable not to introduce substitutions or deletions at these sites.

In the polypeptides of the above (II) and (III), when an amino acid substitution is introduced in SEQ ID NO: 1, examples of a preferred aspect of the amino acid substitution introduced include a conservative substitution. That is, examples of the substitution in the polypeptides of the above (II) and (III) include the following substitutions: when an amino acid to be substituted is a non-polar amino acid, a substitution with other non-polar amino acids; when an amino acid to be substituted is a non-charged amino acid, a substitution with other non-charged amino acids; when an amino acid to be substituted is an acidic amino acid, a substitution with other acidic amino acids; and when an amino acid to be substituted is a basic amino acid, a substitution with other basic amino acids.

From the viewpoint of further improving the specific activity in a temperature region higher than the optimum temperature (60°C) of the protein glutaminase derived from C. proteolyticum consisting of the amino acid sequence set forth in SEQ ID NO: 1, more preferably from the viewpoint of further improving the specific activity in the higher temperature region in the case of using a protein as a substrate, preferred examples of the polypeptides of the above (II) and (III) include polypeptides in which a substitution of the amino acid residue (serine residue) at position 26 in the amino acid sequence set forth in SEQ ID NO: 1 to a proline residue (hereinafter, also referred to as "predetermined other substitution") is introduced in addition to the predetermined substitution, and more preferred examples include polypeptides containing all of the predetermined substitutions (all of the four substitutions) and the predetermined other substitution.

The polypeptides shown in the above (I) to (III) have protein glutaminase activity, and have an optimum temperature in a temperature region higher than the optimum temperature (60°C) of the protein glutaminase derived from C. proteolyticum consisting of the amino acid sequence set forth in SEQ ID NO: 1. The specific optimum temperature of the polypeptides shown in (I) to (III) is an optimum temperature when Z-Gln-Gly (benzyloxycarbonyl-L-glutaminylglycine) is used as a substrate, and is, for example, 61 to 70°C, preferably 62 to 68°C, further preferably 63 to 67°C, and still more preferably 64 to 66°C.

The modified protein glutaminase of the present invention may be used as an active ingredient of an enzyme preparation described later, or may be used for forming a part of a larger protein integrated in the form of a fusion protein or the like with peptides or proteins consisting of other amino acid sequences (hereinafter, also referred to as "other proteins etc."). Examples of the other proteins etc. include peptides used for purification of proteins, such as a polyhistidine residue, and derived from an additional sequence for securing stability of mRNA during recombinant production.

### 2. DNA

A DNA of the present invention is a DNA encoding the modified protein glutaminase described in the above "1. Modified protein glutaminase".

The DNA of the present invention is not particularly limited as long as it is a DNA including a nucleotide sequence encoding a modified protein glutaminase consisting of the polypeptides shown in (I) to (III) described in the above "1. Modified protein glutaminase". Examples of the nucleotide sequence of the DNA encoding the amino acid sequence set forth in SEQ ID NO: 1 (protein glutaminase derived from Chryseobacterium proteolyticum), which is a reference sequence for the polypeptides shown in (I) to (III), include SEQ ID NO: 3. Therefore, the DNA of the present invention can be appropriately designed by those skilled in the art using SEQ ID NO: 3 as a reference sequence.

Examples of the DNA of the present invention include DNAs shown in any one of the following [i] to [iii]:
[i] a DNA consisting of a nucleotide sequence obtained by introducing at least one of a substitution at positions 4 to 6 with a nucleotide sequence encoding a valine residue, a substitution at positions 337 to 339 with a nucleotide sequence encoding an alanine residue, a substitution at positions 460 to 462 with a nucleotide sequence encoding an aspartic acid residue, and a substitution at positions 472 to 474 with a nucleotide sequence encoding a histidine residue, into the nucleotide sequence set forth in SEQ ID NO: 3; [ii] a DNA encoding a polypeptide in which the optimum temperature is higher than the optimum temperature of the polypeptide consisting of the amino acid sequence set forth in SEQ ID NO: 1, the DNA hybridizing under stringent conditions with a DNA consisting of a nucleotide sequence complementary to the DNA shown in the above [i]; and [iii] a DNA encoding a polypeptide in which the optimum temperature is higher than the optimum temperature of the polypeptide consisting of the amino acid sequence set forth in SEQ ID NO: 1, the DNA having a homology of 70% or more with the DNA shown in the above [i].

The specific sequence of the DNA of the above [i] is readily determined by those skilled in the art, and examples of the sequence of DNA encoding a polypeptide consisting of the amino acid sequence set forth in SEQ ID NO: 2 in which all of the four substitutions of the preferred embodiment have been introduced include SEQ ID NO: 4.

For the DNA of [ii] above, the expression "under stringent conditions" refers to conditions of incubating in 6×SSC (1×SSC is 0.15 M NaCl, 0.015 M sodium citrate, pH 7.0) containing 0.5% SDS, 5×Denhartz's [Denhartz's, 0.1% bovine serum albumin (BSA), 0.1% polyvinylpyrrolidone, 0.1% Ficoll 400], and 100 µg/ml of a salmon sperm DNA at 50°C to 65°C for 4 hours to overnight.

The hybridization under stringent conditions is specifically performed by the following method. That is, a nylon membrane on which a DNA library or a cDNA library is immobilized is prepared, and the nylon membrane is blocked at 65°C in a prehybridization solution containing 6×SSC, 0.5% SDS, 5×Denhartz's, and 100 µg/ml of a salmon sperm DNA. Thereafter, each probe labeled with ³²P is added and incubated at 65°C overnight. This nylon membrane is washed in 6×SSC at room temperature for 10 minutes, in 2×SSC containing 0.1% SDS at room temperature for 10 minutes, and in 0.2×SSC containing 0.1% SDS at 45°C for 30 minutes, then autoradiography is performed, and a DNA specifically hybridized with the probe can be detected.

The homology of the DNA of [iii] above may be 70% or more, and is preferably 80% or more, or 85% or more, more preferably 90% or more or 93% or more, further preferably 95% or more, even more preferably 98% or more, still more preferably 98.5% or more or 99% or more, and particularly preferably 99.3% or more or 99.5% or more.

Here, the "homology" of DNA is calculated using a disclosed or commercially available software with an algorithm that makes a comparison using the reference sequence as a query sequence. Specifically, BLAST, FASTA, or GENETYX (manufactured by GENETYX K.K.), or the like can be used, and these may be used with default parameters being set.

The DNA of the present invention can be obtained, for example, by introducing the predetermined substitution into a DNA encoding a polypeptide (i.e., protein glutaminase derived from C. proteolyticum) consisting of the amino acid sequence set forth in SEQ ID NO: 1. The DNA of the present invention can also be artificially synthesized by a total gene synthesis method.

For obtaining the DNA encoding a polypeptide consisting of the amino acid sequence set forth in SEQ ID NO: 1, from a nucleic acid construct such as a plasmid in which the nucleotide sequence set forth in SEQ ID NO: 3 is incorporated, the nucleotide sequence portion can be acquired by a conventional method using PCR. Note that, together with the nucleotide sequence set forth in SEQ ID NO: 3, a sequence obtained by adding a signal sequence and pro-sequence to the 5'-terminus side of the nucleotide sequence can be incorporated into the nucleic acid construct.

As the method for artificially modifying an amino acid sequence by introducing a mutation into a gene, known methods such as a Kunkel method and a Gapped duplex method, and a mutation introduction kit using a site-directed mutagenesis method, for example, QuikChange^{™} Site-Directed Mutagenesis Kit (Stratagene), GeneTailor^{™} Site-Directed Mutagenesis System (Invitrogen), TaKaRa Site-Directed Mutagenesis System (Mutan-K, Mutan-Super Express Km, etc.: Takara Bio Inc.), and the like can be used.

The DNA of the present invention includes various kinds of DNA derived from codon degeneracy. Artificial production of various kinds of DNA encoding the same amino acid sequence can be easily performed using a known genetic engineering method. For example, in genetic engineering production of protein, the expression level of a protein of interest may be low when the frequency of use of a codon used on an original gene encoding the protein is low in the host. In this case, high expression of the protein of interest can be achieved by optimizing the codon usage frequency for the host without changing the encoded amino acid sequence.

As an index representing the codon usage frequency, the total host optimal codon usage frequency of each codon may be adopted. The optimal codon is defined as a codon having the highest usage frequency among codons corresponding to the same amino acid. The codon usage frequency is not particularly limited as long as it is optimized for the host, and examples of the optimal codon for Escherichia coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tge), W: tryptophan (tgg), R: arginine (cgc), G: glycine (ggc).

The nucleotide sequence of DNA in which a mutation is introduced into a nucleotide sequence can be confirmed by sequencing by a conventional method. Specific examples of the sequencing method include a dideoxynucleotide chain termination method (Sanger et al. (1977) Proc. Natl. Acad. Sci. USA 74: 5463), and a sequence analysis method using an appropriate DNA sequencer. Examples of the method for confirming whether DNA is DNA encoding a polypeptide of interest include a method in which a sequenced nucleotide sequence is compared with an unsubstituted nucleotide sequence such as the nucleotide sequence set forth in SEQ ID NO: 3, and a method in which an amino acid sequence deduced from the sequenced nucleotide sequence is compared with an unsubstituted amino acid sequence such as the amino acid sequence set forth in SEQ ID NO: 1.

### 3. Expression cassette or recombinant vector

An expression cassette or recombinant vector of the present invention includes the DNA of the present invention described in the above "2. DNA". The expression cassette or recombinant vector of the present invention can be obtained by linking a promoter and a terminator to the DNA of the present invention, or by inserting the expression cassette of the present invention or the DNA of the present invention into an expression vector.

The expression cassette of the present invention or the recombinant vector of the present invention may further include, as control factors, transcription elements such as an enhancer, a CCAAT box, a TATA box, and an SPI site, in addition to promoter and a terminator. These control factors may be operably linked to the DNA of the present invention. The expression "operably linked" means that various control factors that control the DNA of the present invention are linked to the DNA of the present invention in a state of being capable of operating in a host cell.

Regarding the recombinant vector of the present invention, as the expression vector, those constructed for genetic recombination from phage, plasmid, or virus capable of autonomous replication in the host are suitable. Such expression vectors are well-known, and examples of a commercially available expression vector include pQE vector (QIAGEN GmbH), pDR540 and pRIT2T (GE Healthcare Bio-Sciences KK), and pET vector (Merck KGaA, Darmstadt, Germany). The expression vector may be used by selecting an appropriate combination with a host cell, and for example, in the case of using Escherichia coli as a host cell, a combination of the pET vector and an Escherichia coli strain DH5α, a combination of the pET vector and an Escherichia coli strain BL21 (DE3), a combination of the pDR540 vector and an Escherichia coli strain JM109, or the like is mentioned.

### 4. Transformant

A transformant of the present invention is obtained by transforming a host with the expression cassette or recombinant vector of the present invention described in the above "3. Expression cassette or recombinant vector".

The host used for the production of the transformant of the present invention is not particularly limited as long as the gene can be introduced, the expression cassette or recombinant vector is stable and capable of autonomous replication, and a trait of a gene including the DNA of the present invention can be expressed, but for example, bacteria belonging to the genus Escherichia such as Escherichia coli, the genus Bacillus such as Bacillus subtilis, the genus Pseudomonas such as Pseudomonas putida, and the genus Chryseobacterium such as Chryseobacterium proteolyticum; yeasts, and the like are preferably exemplified, and other than, the host may be an animal cells, an insect cell, a plant, and the like.

The transformant of the present invention can be obtained by introducing the expression cassette of the present invention or the recombinant vector of the present invention into a host. The place where the DNA of the present invention is introduced is also not particularly limited as long as a target gene can be expressed, and may be on a plasmid or on a genome. Specific examples of the method for introducing the expression cassette of the present invention or the recombinant vector of the present invention include a recombinant vector method and a genome editing method. Conditions for introducing the expression cassette or the recombinant vector into the host may be appropriately set according to the type of the host, and the like. When the host is a bacterium, for example, a method using a competent cell by a calcium ion treatment, an electroporation method, and the like are mentioned. When the host is a yeast, for example, an electroporation method, a spheroplast method, a lithium acetate method, and the like are mentioned. When the host is an animal cell, for example, an electroporation method, a calcium phosphate method, a lipofection method, and the like are mentioned. When the host is an insect cell, for example, a calcium phosphate method, a lipofection method, an electroporation method, and the like are mentioned. When the host is a plant cell, for example, an electroporation method, an Agrobacterium method, a particle gun method, a PEG method, and the like are mentioned.

Whether the expression cassette of the present invention or the recombinant vector of the present invention has been incorporated into a host can be confirmed by a PCR method, a Southern hybridization method, a Northern hybridization method, and the like.

When it is confirmed whether the expression cassette of the present invention or the recombinant vector of the present invention has been incorporated into a host by a PCR method, for example, the genome DNA, the expression cassette, or the recombinant vector may be separated and purified from a transformant.

For example, when the host is a bacterium, the separation and purification of the expression cassette or the recombinant vector are performed based on a lysate obtained by lysing the bacterium. As a lysis method, for example, a treatment is performed with a lytic enzyme such as lysozyme, and as necessary, a protease, other enzymes, and a surfactant such as sodium lauryl sulfate (SDS) are used in combination.

Physical crushing methods such as freeze thawing and French press processing may be combined. The separation and purification of DNA from a lysate can be performed, for example, by appropriately combining a deproteinization treatment using a phenol treatment and a protease treatment, a ribonuclease treatment, an alcohol precipitation treatment, and a commercially available kit.

DNA cleavage can be performed according to a conventional method, for example, using a restriction enzyme treatment. As a restriction enzyme, for example, a type II restriction enzyme that acts on a specific nucleotide sequence is used. Binding between the DNA and the expression cassette or the expression vector is performed, for example, using a DNA ligase.

Thereafter, PCR is performed by designing a primer specific to the DNA of the present invention using the separated and purified DNA as a template. The amplification product obtained by PCR is subjected to agarose gel electrophoresis, polyacrylamide gel electrophoresis, capillary electrophoresis, or the like, and is stained with ethidium bromide, SYBR Green solution, and the like, and then the amplification product is detected as a band, so that transformation can be confirmed.

PCR can be performed using a primer labeled with a fluorescent dye or the like in advance to detect an amplification product. A method of binding an amplification product to a solid phase such as a microplate and confirming the amplification product by fluorescence, an enzyme reaction, or the like may also be adopted.

### 5. Method for producing modified protein glutaminase

A method for producing a modified protein glutaminase of the present invention is a method for producing the enzyme described in the above "1. Modified protein glutaminase" and includes a step of culturing the transformant of the present invention. Note that, when the predetermined substitution contained in the modified protein glutaminase is naturally introduced, the modified protein glutaminase can be obtained by a production method including a step of culturing a microorganism producing the modified protein glutaminase.

The culture conditions may be appropriately set in consideration of the nutritional physiological properties of the transformant or the microorganism, and liquid culture is preferably mentioned. **In** the case of performing industrial production, aeration-agitation culture is preferred. As a nutrient source of the medium, those required for growth of the transformant or the microorganism can be used. A carbon source may be any assimilable carbon compound, and examples thereof include glucose, sucrose, lactose, maltose, molasses, and pyruvic acid. A nitrogen source may be any assimilable nitrogen compound, and examples thereof include peptone, meat extract, yeast extract, casein hydrolysate, and soybean cake alkaline extract. **In** addition to the carbon source and the nitrogen source, for example, phosphates, carbonates, sulfates, salts of magnesium, calcium, potassium, iron, manganese, zinc, and the like, specific amino acids, specific vitamins, and the like may be used as necessary.

The culture temperature can be appropriately set as long as the transformant of the present invention or the microorganisms can grow, and the transformant or the microorganisms produce a modified protein glutaminase, and is preferably about 15 to 37°C. The culturing may be completed at an appropriate time with reference to the time when the modified protein glutaminase reaches the highest yield, and the culturing time is usually about 12 to 48 hours.

After the transformant or the microorganisms are cultured, culture supernatant and/or the microbial cells are recovered by subjecting the culture broth to a method such as centrifugation. The microbial cells are treated by a mechanical method such as ultrasonic wave and French press or a lytic enzyme such as lysozyme, and as necessary, are solubilized by using an enzyme such as protease or a surfactant such as sodium lauryl sulfate (SDS), whereby a water-soluble fraction containing a predetermined modified protein glutaminase can be obtained. By selecting an appropriate expression cassette or expression vector and a host, the expressed modified protein glutaminase can also be secreted into the culture broth.

The water-soluble fraction containing the modified protein glutaminase obtained as described above may be subjected to a purification treatment as it is, but may be subjected to a purification treatment after the modified protein glutaminase in the water-soluble fraction is concentrated. The concentration can be performed by, for example, concentration under reduced pressure, membrane concentration, a salting-out treatment, fractionation precipitation with a hydrophilic organic solvent (for example, methanol, ethanol, acetone, and the like), or the like.

The purification treatment of the modified protein glutaminase can be performed, for example, by appropriately combining methods such as gel filtration, adsorption chromatography, ion exchange chromatography, and affinity chromatography. The purified modified protein glutaminase may be powderized by lyophilization, vacuum drying, spray drying, or the like if necessary.

### 6. Enzyme preparation

The modified protein glutaminase can be provided in the form of an enzyme preparation. Therefore, the present invention also provides an enzyme preparation containing the modified protein glutaminase described in the above "1. Modified protein glutaminase" as an active ingredient.

The content of the modified protein glutaminase in the enzyme preparation of the present invention is not particularly limited, but the lower limit of the content is, for example, 1 U/g or more, 5 U/g or more, or 10 U/g or more, and preferably 50 U/g or more, 100 U/g or more, 500 U/g or more, or 800 U/g or more. The upper limit of the content is, for example, 10000 U/g or less, 5000 U/g or less, 2000 U/g or less, 1500 U/g or less, or 1000 U/g or less. The specific contents include 1 to 10000 U/g, 5 to 10000 U/g, 10 to 10000 U/g, 50 to 5000 U/g, 100 to 2000 U/g, 500 to 1500 U/g, or 800 to 1000 U/g.

Note that, for the protein glutaminase activity, Z-Gln-Gly (benzyloxycarbonyl-L-glutaminylglycine) is used as a substrate, and the enzyme activity of producing 1 µmol of ammonia per minute is defined as 1 unit (1 U).

The enzyme preparation of the present invention may contain other components to the extent that the effect of the present invention is not affected, in addition to the modified protein glutaminase. Examples of the other components include enzymes other than the modified protein glutaminase, additives, and culture residues generated by the production method.

Examples of the other enzymes include amylase (a-amylase, β-amylase, or glucoamylase), glucosidase (a-glucosidase or β-glucosidase), galactosidase (α-galactosidase or β-galactosidase), protease (acidic protease, neutral protease, or alkaline protease), peptidase (leucine peptidase or aminopeptidase), lipase, esterase, cellulase, phosphatase (acid phosphatase or alkaline phosphatase), nuclease, deaminase, oxidase, dehydrogenase, glutaminase, pectinase, catalase, dextranase, transglutaminase, protein deamidase (other than the above-described modified protein glutaminase), and pullulanase. These other enzymes may be contained singly or in combination of a plurality of kinds thereof.

Examples of the additives include an excipient, a buffer agent, a suspending agent, a stabilizer, a preservative, an antiseptic, and physiological saline. Examples of the excipient include starch, dextrin, maltose, trehalose, lactose, D-glucose, sorbitol, D-mannitol, sucrose, and glycerol. Examples of the buffer agent include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic include ethanol, benzalkonium chloride, para-hydroxybenzoic acid, and chlorobutanol. These additives may be contained singly or in combination of a plurality of kinds thereof.

Examples of the culture residues include components derived from a culture medium, contaminant proteins, and microbial cell components.

The form of the enzyme preparation of the present invention is not particularly limited, and examples thereof include a liquid form and a solid form (such as powder or granules). The enzyme preparation in these forms can be prepared by a generally known method.

### 7. Modifier for protein material

The modified protein glutaminase can be used for known applications of protein glutaminase. For example, the modified protein glutaminase can be used for the purpose of modifying a protein material. Therefore, the present invention also provides a modifier for a protein material, the modifier containing the modified protein glutaminase.

A specific aspect of the modification of the protein material is not particularly limited, and any modification may be a change in properties of the protein caused by generation of a carboxyl group by deamidation of a γ-amide group and a β-amide group of a glutamine residue and an asparagine residue of the protein. Specifically, examples of the modification of the protein material include an increase in solubility of the protein, an increase in water dispersibility, an improvement in emulsifying power, an improvement in emulsion stability, an improvement in foaming property, and an improvement in foam stability. A specific method of using the modifier for a protein material is as described in "8. Method for producing modified protein material" below.

### 8. Method for producing modified protein material

As described above, the modified protein glutaminase can be used for the purpose of modifying a protein material. Therefore, the present invention also provides a method for producing a modified protein material, including an enzyme treatment step of allowing the modified protein glutaminase to act on a protein material.

In the enzyme treatment step, for example, a mixture containing a protein material and a modified protein glutaminase is placed under the action conditions of the modified protein glutaminase so that a protein modifying reaction can proceed.

The protein material is not particularly limited as long as it is a material containing a protein, and may be either one for edible use or one for non-food use. The edible protein material can be used as food and drink or as a material for producing food and drink. The non-food protein material can be used as a material for protein experiments, a medical material, a fiber material, a cosmetic material, or the like.

Specific examples of the protein material include protein source itself (i.e., an organ of an organism, a part thereof, a body fluid of an organism, or the like), and a preparation obtained by performing treatment for concentrating a protein by removing at least one component other than a protein from the protein source by a known method, and these are appropriately selected by those skilled in the art. The protein may be either a plant protein or an animal protein, and either one of them may be used, or both may be used in combination.

When the protein is a plant protein, examples of the plant protein include proteins of pulses such as soybean, pea, lentil bean, chickpea, black bean, fava bean, mung bean, lupine bean, and kidney bean; proteins of cereal crops such as wheat, barley, oat, sorghum, rice, rye, buckwheat, Japanese barnyard millet, foxtail millet, teff, corn, and potato; and proteins of seeds such as almond, coconut, peanut, cashew nut, hazelnut, pecan nut, macadamia nut, pistachio, walnut, brazil nut, pili nut, chestnut, sesame, pine nut, hemp seed (the term "hemp" refers to industrial hemp containing less than 0.3 wt%, preferably less than 0.2 wt%, and more preferably less than 0.01 wt% of tetrahydrocannabinol per dry weight, and most preferably containing no tetrahydrocannabinol), chia seed, quinoa, Amaranthus, canary seed, and linseed. These plant proteins may be used singly or in combination of two or more kinds thereof. Among these plant proteins, soybean, pea, lentil bean, chickpea, fava bean, mung bean, wheat, oat, rice, and almond are preferable, soybean, pea, lentil bean, chickpea, fava bean, mung bean, wheat, oat, and almond are more preferable, soybean, lentil bean, chickpea, fava bean, mung bean, wheat, oat, and almond are further preferable, and soybean, fava bean, mung bean, wheat, oat, and almond are still more preferable.

When the protein is an animal protein, examples of the animal protein include proteins of livestock meat, fish meat, eggs, milk, serum, hair, insects, and the like, for example, casein (such as α-casein, β-casein, or κ-casein), whey protein, albumin (such as ovalbumin, α-lactalbumin, or serum albumin), globulin (such as β-lactoglobulin or immunoglobulin), lactoferrin, wool, silk, myofibrillar protein (such as myosin, actin, troponin, or tropomyosin), and muscle matrix protein (such as collagen). These animal proteins may be used singly or in combination of two or more kinds thereof. Among these animal proteins, egg protein and milk protein are preferable, casein, whey protein, and ovalbumin are further preferable, and casein is still more preferable.

The form of the protein material may be untextured or textured. When the form of the protein material is untextured, examples of the form include flowable forms such as a liquid form, a slurry form, and a paste form.

When the protein material is an untextured protein material containing a plant protein, the protein material can be used in the form of the following plant protein-containing liquid composition at the time of treatment with a modified protein glutaminase: (i) a liquid obtained by using a dry powder of a plant raw material (an organ of an origin plant of a plant protein or a part thereof) as a plant material, dispersing the plant material or a water-soaked material thereof in water, and as necessary, removing a water-insoluble matter derived from the plant raw material by any means such as centrifugal filtration, filtration, a filter bag, or a sieve; (ii) a liquid obtained by using a plant raw material itself or a dried product thereof as a plant material, crushing and dispersing the plant material or a water-soaked material thereof in water, and as necessary, removing a water-insoluble matter derived from the plant raw material by any means such as centrifugal filtration, filtration, a filter bag, or a sieve; (iii) a liquid in which the plant protein content is increased by, for example, removing at least a part of a component other than the plant protein from a liquid of (i) or (ii) described above; and (iv) a liquid obtained by using a dry powder prepared from any liquid of (i) to (iii) described above as a plant material, and dissolving and/or dispersing the plant material or a water-soaked material thereof in water.

A protein glutaminase without modification (i.e., protein glutaminase derived from C. proteolyticum) maintains a relatively high protein glutaminase activity even in a temperature region higher than the optimum temperature by about 5°C when an animal protein is used as a substrate, but the activity rapidly decreases in a temperature region higher than the optimum temperature by about 5°C when a low-molecular-weight substance such as Z-Gln-Gly and a plant protein are used as substrates. On the other hand, when a plant protein is used as a substrate, the modified protein glutaminase of the present invention can maintain a relatively high protein glutaminase activity even in a temperature region higher than the optimum temperature by about 5°C. For this reason, even on a plant protein in which protein glutaminase activity has not been capable of being exerted so far in a treatment in a high temperature region (for example, about 70°C, specifically, 67 to 73°C, 68 to 72°C, or 69 to 71°C), a remarkably high activity can be exerted. From such a viewpoint, a suitable protein material includes a material containing a plant protein.

When the substrate is an animal protein, the optimum temperature of the modified protein glutaminase of the present invention may be increased as compared with a case where the substrate is a plant protein. Therefore, in a preferred embodiment of the present invention, a material containing an animal protein that enables protein treatment in a higher temperature region (for example, 65 to 77°C, 65 to 76°C, or 65 to 75°C) is used.

The content of the protein contained in these protein materials is not particularly limited, and is, for example, 10 to 100 wt%, 20 to 100 wt%, 30 to 100 wt%, 40 to 100 wt%, or 50 to 100 wt%. The lower limit of the range of the content of these proteins may be, for example, 40 wt% or more, 45 wt% or more, 50 wt% or more, 55 wt% or more, 60 wt% or more, 70 wt% or more, 80 wt% or more, or 90 wt% or more. The upper limit of the range of the content of these proteins may be, for example, 95 wt% or less, 90 wt% or less, 85 wt% or less, 80 wt% or less, 70 wt% or less, or 60 wt% or less.

Examples of the content of the protein material in the mixture include an amount such that the concentration of protein contained in the protein material in the mixture is, for example, 0.01 wt% or more, 0.05 wt% or more, 0.1 wt% or more, 0.5 wt% or more, or 1 wt% or more, preferably 2 wt% or more, or 3 wt% or more, and more preferably 4 wt% or more. The upper limit of the concentration of protein contained in the protein material in the mixture is not particularly limited, and is, for example, 80 wt% or less, 60 wt% or less, 40 wt% or less, 30 wt% or less, 20 wt% or less, 15 wt% or less, 10 wt% or less, 8 wt% or less, or 5.5 wt% or less. Specific ranges of the content of the protein material in the mixture include 0.01 to 80 wt%, 0.05 to 60 wt%, 0.1 to 40 wt%, 0.5 to 30 wt%, or 1 to 20 wt%, preferably 2 to 15 wt%, or 3 to 10 wt%, and more preferably 4 to 8 wt%, or 4 to 5.5 wt%.

The used amount of the modified protein glutaminase is not particularly limited, and the used amount of the modified protein glutaminase per 1 g of protein contained in the protein material is, for example, 0.01 U or more, or 0.1 U or more, preferably 0.5 U or more, more preferably 1 U or more, further preferably 2 U or more, still more preferably 3.5 U or more, and even more preferably 4.5 U or more. The upper limit of the used amount of the modified protein glutaminase per 1 g of protein contained in the protein material is not particularly limited, and is, for example, 50 U or less, 45 U or less, 35 U or less, 20 U or less, 10 U or less, 8 U or less, or 5.5 U or less.

The used amount of the modified protein glutaminase per 1 g of the protein material is, for example, 0.01 U or more, or 0.1 U or more, preferably 0.5 U or more, more preferably 1 U or more, further preferably 2 U or more, still more preferably 3 U or more, and even more preferably 4 U or more. The upper limit of the used amount of the modified protein glutaminase per 1 g of the protein material is not particularly limited, and is, for example, 50 U or less, 40 U or less, 30 U or less, 20 U or less, 10 U or less, 7 U or less, or 5 U or less.

The action conditions of the modified protein glutaminase (such as temperature condition and pH condition) are appropriately determined based on the optimum temperature, optimum pH, and the like of the protein glutaminase to be used.

The optimum temperature of the modified protein glutaminase is higher than 60°C, which is the optimum temperature of protein glutaminase that is a polypeptide consisting of the amino acid sequence set forth in SEQ ID NO: 1 and does not contain the predetermined substitution. Therefore, in the case of acting on a plant protein, the temperature condition is, for example, 61°C or higher, preferably 62°C or higher, further preferably 63°C or higher, and still more preferably 64°C or higher, or 65°C or higher, and the upper limit thereof is, for example, 70°C or lower, 69°C or lower, 68°C or lower, 67°C or lower, or 66°C or lower. In the case of acting on an animal protein, a suitable temperature condition is 66°C or higher, 67°C or higher, 68°C or higher, 69°C or higher, or 70°C or higher, and the upper limit thereof is, for example, 77°C or lower, 76°C or lower, 75°C or lower, 74°C or lower, 73°C or lower, 72°C or lower, or 71°C or lower.

Furthermore, when a protein is used as a substrate, the modified protein glutaminase can maintain a relatively high enzyme activity even at a temperature higher than the optimum temperature by, for example, about 5°C as compared with the case of using Z-Gln-Gly (benzyloxycarbonyl-L-glutaminylglycine) as a substrate. Therefore, in the case of acting on a plant protein, the temperature condition is even more preferably 66°C or higher, 67°C or higher, 68°C or higher, 69°C or higher, or 70°C or higher as well as the above-described temperature condition (i.e., 61°C or higher, preferably 62°C or higher, further preferably 63°C or higher, and still more preferably 64°C or higher, or 65°C or higher), and the upper limit thereof is, for example, 73°C or lower, 72°C or lower, or 71°C or lower as well as the above-described temperature condition (i.e., 70°C or lower, 69°C or lower, 68°C or lower, 67°C or lower, or 66°C or lower). In the case of acting on an animal protein, the temperature condition is 71°C or higher, 72°C or higher, 73°C or higher, 74°C or higher, or 75°C or higher as well as the above-described temperature condition (i.e., 66°C or higher, 67°C or higher, 68°C or higher, 69°C or higher, or 70°C or higher), and the upper limit thereof is, for example, 80°C or lower, 79°C or lower, 78°C or lower, 77°C or lower, or 76°C or lower as well as the above-described temperature condition (i.e., 77°C or lower, 76°C or lower, 75°C or lower, 74°C or lower, 73°C or lower, 72°C or lower, or 71°C or lower).

Therefore, specific ranges of the temperature condition under which the modified protein glutaminase is allowed to act are, in the case of acting on a plant protein, for example, 61 to 73°C, 62 to 73°C, 63 to 73°C, 64 to 73°C, 65 to 73°C, 66 to 73°C, 67 to 73°C, 68 to 73°C, 69 to 73°C, 70 to 73°C, 61 to 72°C, 61 to 71°C, 61 to 70°C, 61 to 69°C, 61 to 68°C, 61 to 67°C, or 61 to 66°C; and in the case of acting on an animal protein, 66 to 80°C, 67 to 80°C, 68 to 80°C, 69 to 80°C, 70 to 80°C, 71 to 80°C, 72 to 80°C, 73 to 80°C, 74 to 80°C, 75 to 80°C, 66 to 79°C, 66 to 78°C, 66 to 77°C, 66 to 76°C, 66 to 75°C, 66 to 74°C, 66 to 73°C, 66 to 72°C, or 66 to 71°C.

Among the action conditions of the modified protein glutaminase, as the pH condition, the pH at 25°C is, for example, 2 to 12, preferably 3 to 10, more preferably 4 to 9, further preferably 5.5 to 7, and still more preferably 6 to 7.

The time for which the modified protein glutaminase is allowed to act is not particularly limited, and may be appropriately determined according to the preparation scale or the like, and is, for example, 10 minutes or longer, 30 minutes or longer, 1 hour or longer, 8 hours or longer, 16 hours or longer, or 20 hours or longer. The upper limit of the range of the time is not particularly limited, and is, for example, 40 hours or shorter, 30 hours or shorter, 25 hours or shorter, 20 hours or shorter, 10 hours or shorter, 8 hours or shorter, 6 hours or shorter, 4 hours or shorter, 2 hours or shorter, or 1 hour or shorter. Specific ranges of the time for which the modified protein glutaminase is allowed to act include 10 minutes to 40 hours, 30 minutes to 40 hours, 1 to 40 hours, 8 to 40 hours, 16 to 40 hours, 20 to 40 hours, 10 minutes to 30 hours, 10 minutes to 25 hours, 10 minutes to 20 hours, 10 minutes to 10 hours, 10 minutes to 8 hours, 10 minutes to 6 hours, 10 minutes to 4 hours, 10 minutes to 2 hours, or 10 minutes to 1 hour.

The protein material can be treated not only with the modified protein glutaminase but also with an enzyme other than the modified protein glutaminase as necessary. The treatment with another enzyme may be performed simultaneously with the treatment with the modified protein glutaminase, or may be performed before or after the treatment with the modified protein glutaminase. When the optimum temperature of the other enzyme overlaps with the optimum temperature range of the modified protein glutaminase, in the enzyme treatment step, the treatment with the modified protein glutaminase and the treatment with the other enzyme can be performed simultaneously. **In** this treatment, both the treatment with the modified protein glutaminase and the treatment with the other enzyme can be performed under a common temperature condition. Specific temperature conditions can be selected from the temperature conditions described above.

For example, when the protein material is used in the form of the above-described plant protein-containing liquid composition, and the plant protein-containing liquid composition is prepared using cereal crops as a plant raw material, for example, an amylase can be used as another enzyme. The amylase can be used for the purpose of improving the solubility of a plant protein-containing liquid composition prepared using cereal crops. Examples of the amylase include α-amylase and β-amylase. The α-amylase is not particularly limited, but examples thereof include α-amylases derived from the genus Aspergillus and the genus Bacillus, and α-amylases derived from the genus Bacillus, Bacillus subtilis, Bacillus amyloliquefaciens, and Bacillus licheniformis are preferable. The β-amylase is not particularly limited, but examples thereof include β-amylases derived from a plant (wheat or soybean) and the genus Bacillus, β-amylase derived from the genus Bacillus is preferable, and β-amylase derived from Bacillus flexus species is more preferable. These amylases may be used singly or in combination of two or more kinds thereof. In a preferred embodiment of the present invention, in the enzyme treatment step, a plant protein-containing liquid composition prepared using cereal crops can be simultaneously treated with a modified protein glutaminase and an amylase. In this treatment, both the treatment with the modified protein glutaminase and the treatment with the amylase can be performed under a common temperature condition. Specific temperature conditions can be selected from the above-described temperature conditions (i.e., the temperature condition in the case of acting on a plant protein).

After completion of the reaction, an enzyme inactivation treatment is performed, followed by cooling and, as necessary, post-treatment, whereby a modified protein material is obtained.

Specific examples of the modified protein material include, as those obtained from a material containing an animal protein, milk, cream (such as coffee creamer), a dried product of milk or cream (such as skimmed milk powder), a milk fermented product (such as cheese or yogurt), a meat processed food product (such as livestock meat, chicken, or minced fish processed food product, more specifically, hamburg steak, meat ball, patty, meat loaf, minced cutlet, or dim sum), and include, as those obtained from a material containing a plant protein, plant milk, plant cream (such as plant coffee creamer), a dried product of plant milk or plant cream (such as powdered plant milk), a plant milk fermented product (such as alternative cheese or alternative yogurt), and a meat-like processed food product (referring to a food product imitating livestock meat, chicken and/or minced fish processed food product with substitute meat, and examples thereof include food products imitating hamburg steak, meat ball, patty, meat loaf, minced cutlet, dim sum, or the like with substitute meat).

### Examples

Hereinafter, the present invention will be specifically described by means of Examples; however, the present invention is not to be construed as being limited to the following Examples.

### (1) Preparation of modified protein glutaminase (A2V/T113A/N154D/N158H and A2V/S26P/T113A/N154D/N158H)

Mutations were randomly introduced using, as a template, the nucleotide sequence (SEQ ID NO: 3) encoding the mature amino acid sequence (SEQ ID NO: 1) of wild-type C. proteolyticum-derived protein glutaminase introduced into the pET21 vector, to prepare a library consisting of about 8000 mutant strains.

Specifically, PCR was performed by a conventional method using the following primers and Diversify PCR Random Mutagenesis Kit (manufactured by Takara Bio Inc.) to prepare a gene product into which a random mutation was introduced with respect to wild-type C. proteolyticum-derived protein glutaminase (SEQ ID NO: 1).
(Primer for random mutation)
   Fw: 5'-GATCCTGACAAAAGAAGTAAAAGGGCAAACCAATAAA-3'
(SEQ ID NO: 5)
   Rv: 5'-CTCAGTGGTGGTGGTGGTGGTGCTCGAG-3' (SEQ ID NO: 6)

PCR was performed by a conventional method using the following primers and PrimeSTAR GXL DNA Polymerase (manufactured by Takara Bio Inc.) to prepare a gene product of an expression vector.
(Primer for expression vector)
Fw: 5'-CTCGAGCACCACCACCACCACCACTGAG-3' (SEQ ID NO: 7)
Rv: 5'-TTTATTGGTTTGCCCTTTTACTTCTTTTGTCAGGATC-3' (SEQ ID NO: 8)

The obtained gene products were ligated using In-Fusion HD Cloning Kit (manufactured by Takara Bio Inc.), and then E.coli BL21 (DE3) was transformed by a conventional method to obtain a transformant. The transformant was cultured with shaking in LB medium at 37°C for 16 hours. Thereafter, the transformant was transferred to TB medium and cultured at 37°C for 4 hours, and IPTG (final concentration: 0.5 mM) was added, followed by culturing with shaking at 33°C for 20 hours. The bacterial cells were recovered from the culture broth, and the bacterial cells were lysed with B-PER Bacterial Cell Lysis Reagent (manufactured by Thermo Scientific) by a conventional method, and centrifuged at 15,000 rpm for 10 minutes. The recovered centrifugal supernatant was used as a crude enzyme solution, trypsin was added so as to have a final concentration of 0.05 mg/mL, and the mixture was treated at 37°C for 1 hour to prepare a mutant library.

As a result of screening this mutant library, two strains of mutants in which the optimum temperature was shifted to a temperature region higher than that of the wild-type C. proteolyticum-derived protein glutaminase, specifically, a polypeptide A2V/T113A/N154D/N158H quadruple mutant (Example 1) consisting of an amino acid sequence (SEQ ID NO: 2) in which, in the amino acid sequence set forth in SEQ ID NO: 1, substitution of the amino acid residue at position 2 to a valine residue, a substitution of the amino acid residue at position 113 to an alanine residue, a substitution of the amino acid residue at position 154 to an aspartic acid residue, and a substitution of the amino acid residue at position 158 to a histidine residue have been introduced, and a polypeptide A2V/S26P/T113A/N154D/N158H quintuple mutant (Example 2) consisting of an amino acid sequence in which a substitution of the amino acid residue at position 26 to a proline residue has been further introduced in the quadruple mutant were narrowed down.

The target transformant (mutant) was cultured in 5 mL of TB medium at 37°C for 16 hours. 1 mL of the culture broth was inoculated into 200 mL of TB medium, and culture was performed at 27°C for 48 hours. When 24 hours of culture had elapsed, IPTG having a final concentration of 0.1 mM was added. After completion of the culture, the bacterial cells were recovered, and the bacterial cells were crushed with glass beads. After the crushing treatment, centrifugation was performed at 15,000 rpm for 10 minutes. The recovered centrifugal supernatant was used as a crude enzyme solution, trypsin was added so as to have a final concentration of 0.05 mg/mL, and the mixture was treated at 37°C for 1 hour and then purified by a conventional method using TALON^{(R)} SpinColumns (manufactured by Takara Bio Inc.). The obtained enzyme solution was subjected to buffer exchange into a 50 mM phosphate buffer (pH 6.5) using Prepacked Disposable PD-10 Columns (manufactured by Cytiva).

### (2) Protein content test method-BCA method (bicinchoninic acid method)

Pierce BCA Protein Assay Kits (manufactured by Pierce) were used. 50 mL of BCA Protein Assay Reagent (Reagent A Pierce) and 1 mL of BCA Protein Assay Reagent (Reagent B Pierce) were put into a 50 mL stoppered conical flask and mixed to prepare a BCA reagent.

4 mL of a BCA reagent was weighed in a test tube (15 × 150 mm), and allowed to stand at 37°C for exactly 10 minutes, then 0.2 mL of a sample solution containing a protein glutaminase was added thereto, and the mixture was immediately shaken. This solution was allowed to stand at 37°C for exactly 30 minutes, and then cooled with running water. The absorbance (AT) of this solution at a wavelength of 562 nm was measured using water as a control. Separately, 4 mL of the BCA reagent was allowed to stand at 37°C for exactly 10 minutes, then 0.2 mL of water was added thereto, and the mixture was shaken, and then the absorbance (AB) was measured in the same manner as described above. A calibration curve representing the relationship between albumin concentration and the absorbance (562 nm) was prepared.

The protein concentration (mg/g or mg/mL) in the sample solution was calculated from the calibration curve prepared with albumin and the absorbance difference (AT - AB) of the sample solution. [Mathematical Formula 1] $Protein amount \left(mg/g or mL\right) = Tmg \times n$
n: dilution factor per 1 g of sample
T: value calculated from calibration curve

### [Test Example 11 Reactivity to Z-Gln-Gly

### [1-1]

Z-Gln-Gly (benzyloxycarbonyl-L-glutaminylglycine) was used as a substrate, and for the modified protein glutaminase A2V/T113A/N154D/N158H of the present invention (Example 1, SEQ ID NO: 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured.

Specifically, to 1 mL of a 0.2 M phosphate buffer (pH 6.5) containing 30 mM Z-Gln-Gly, 0.1 mL of a sample solution containing a protein glutaminase was added, the mixture was left to stand at each test temperature for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein glutaminase was further added, and the mixture was left to stand at the test temperature for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The enzyme activity of the protein glutaminase was calculated from the following formula with the amount of enzyme that produces 1 µmol of ammonia per minute being defined as 1 unit (1 U). In the formula, the reaction solution amount is 2.1, the enzyme solution amount is 0.1, and Df is a dilution factor of the enzyme solution. 17.03 is a molecular weight of ammonia. [Mathematical Formula 2] 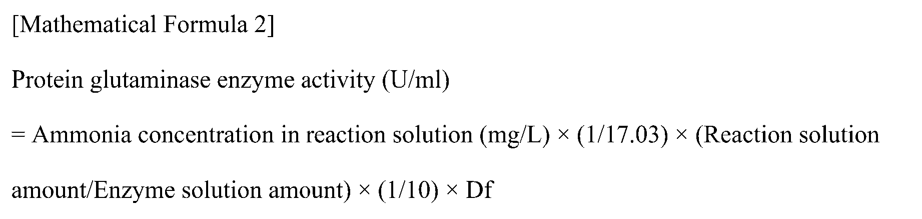 Protein glutaminase enzyme activity (U/ml) = Ammonia concentration in reaction solution (mg/L) × (1/17.03) × (Reaction solution amount/Enzyme solution amount) × (1/10) × Df

The specific activity (U/mg-protein) to Z-Gln-Gly at each test temperature was derived. A relative value of the specific activity (relative activity (%)) at each test temperature when the maximum value among the obtained specific activities was taken as 100% was derived. The results showing the relationship between the relative activity and the temperature are shown in Fig. 1a.

As is apparent from Fig. 1a, the optimum temperature of the modified protein glutaminase A2V/T113A/N154D/N158H of the present invention (Example 1: solid line) for Z-Gln-Gly was 65°C, and was shifted to a temperature region higher than the optimum temperature (60°C) of the wild-type C. proteolyticum-derived protein glutaminase before modification (Comparative Example 1: dashed line) by about 5°C.

### [1-2]

Similarly to the above [1-1], Z-Gln-Gly is used as a substrate, and for the modified protein glutaminase A2V/T113A/N154D/N158H of the present invention (Example 1, SEQ ID NO: 2) and the modified protein glutaminase A2V/S26P/T113A/N154D/N158H of the present invention (Example 2), the specific activity at each test temperature (37°C, 50°C, 60°C, 65°C, 70°C, 75°C, or 80°C) was measured, and the results showing the relationship between the relative value of the specific activity (relative activity (%)) and the temperature are shown in Fig. 1b.

As is apparent from Fig. 1b, the optimum temperature of the modified protein glutaminase A2V/T113A/N154D/N158H of the present invention (Example 1: solid line) and the modified protein glutaminase A2V/S26P/T113A/N154D/N158H of the present invention (Example 2: dashed-dotted line) for Z-Gln-Gly was 65°C. When the results in Fig. 1a are considered together, it is recognized that the modified protein glutaminase of Example 2 was shifted to a temperature region higher than the optimum temperature (60°C) of the wild-type C. proteolyticum-derived protein glutaminase before modification by about 5°C. The modified protein glutaminase of Example 2 had a higher relative activity at 75°C than the modified protein glutaminase of Example 1, and showed a relative activity almost equivalent to that of the modified protein glutaminase of Example 1 at the other test temperatures.

### [Test Example 2] Reactivity to soybean protein

A soybean protein material (product name: Fujipro, manufactured by FUJI OIL CO., LTD., protein content: 86.9 wt%) was used as a substrate, and for the modified protein glutaminase A2V/T113A/N154D/N158H of the present invention (Example 1, SEQ ID NO: 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured.

The test procedure was performed in the same manner as in Test Example 1 except that the substrate was changed to the soybean protein material. At that time, the enzyme was allowed to react so that the final concentration of the soybean protein material was 5 wt%.

The specific activity (U/mg-protein) to soybean protein at each test temperature was derived. A relative value of the specific activity (relative activity (%)) at each test temperature when the maximum value among the obtained specific activities was taken as 100% was derived. The results showing the relationship between the relative activity and the temperature are shown in Fig. 2.

As is apparent from Fig. 2, the optimum temperature of the modified protein glutaminase A2V/T113A/N154D/N158H of the present invention (Example 1: solid line) for soybean protein was 65°C, and was shifted to a temperature region higher than the optimum temperature (60°C) of the wild-type C. proteolyticum-derived protein glutaminase before modification (Comparative Example 1: dashed line) by about 5°C. In the protein glutaminase of Comparative Example 1, the relative activity sharply decreased to 40% in a temperature region (65°C) higher than the optimum temperature (60°C) by 5°C, but in the protein glutaminase of Example 1, a high relative activity of 86% was maintained even at 70°C, which is higher than the optimum temperature (65°C) by 5°C.

### [Test Example 31 Reactivity to pea protein

### [3-1]

A pea protein material (product name: LYSAMINE GPS, manufactured by Roquette Frères, protein content: 85 wt%) was used as a substrate, and for the modified protein glutaminase A2V/T113A/N154D/N158H of the present invention (Example 1, SEQ ID NO: 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured.

The test procedure was performed in the same manner as in Test Example 1 except that the substrate was changed to the pea protein material. At that time, the enzyme was allowed to react so that the final concentration of the pea protein material was 5 wt%.

The specific activity (U/mg-protein) to pea protein at each test temperature was derived. A relative value of the specific activity (relative activity (%)) at each test temperature when the maximum value among the obtained specific activities was taken as 100% was derived. The results showing the relationship between the relative activity and the temperature are shown in Fig. 3a.

As is apparent from Fig. 3a, the optimum temperature of the modified protein glutaminase A2V/T113A/N154D/N158H of the present invention (Example 1: solid line) for pea protein was 65°C, and was shifted to a temperature region higher than the optimum temperature (60°C) of the wild-type C. proteolyticum-derived protein glutaminase before modification (Comparative Example 1: dashed line) by about 5°C. In the protein glutaminase of Comparative Example 1, the relative activity sharply decreased to 40% in a temperature region (65°C) higher than the optimum temperature (60°C) by 5°C, but in the protein glutaminase of Example 1, a high relative activity of 85% was maintained even at 70°C, which is higher than the optimum temperature (65°C) by 5°C.

### [3-2]

Similarly to the above [3-1], the pea protein material is used as a substrate, and for the modified protein glutaminase A2V/S26P/T113A/N154D/N158H of the present invention (Example 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured, and the results showing the relationship between the relative value of the specific activity (relative activity (%)) and the temperature are shown in Fig. 3b.

As is apparent from Fig. 3b, the optimum temperature of the modified protein glutaminase A2V/S26P/T113A/N154D/N158H of the present invention (Example 2: dashed-dotted line) for pea protein was 65°C, and was shifted to a temperature region higher than the optimum temperature (60°C) of the wild-type C. proteolyticum-derived protein glutaminase before modification (Comparative Example 1: dashed line) by about 5°C. As shown in Fig. 3a, the relative activity of the protein glutaminase of Example 1 at 70°C, which is higher than the optimum temperature by 5°C for pea protein, was 85%, and the relative activity of the protein glutaminase of Example 2 was further improved to 90%.

### [Test Example 41 Reactivity to casein

### [4-1]

Milk casein (product name: Casein, Bovine Milk, Carbohydrate and Fatty Acid Free, manufactured by Merck KGaA, Darmstadt, Germany, protein content: 99 wt%) was used as a substrate, and for the modified protein glutaminase A2V/T113A/N154D/N158H of the present invention (Example 1, SEQ ID NO: 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured.

The test procedure was performed in the same manner as in Test Example 1 except that the substrate was changed to the milk casein. At that time, the enzyme was allowed to react so that the final concentration of the milk casein was 5 wt%.

The specific activity (U/mg-protein) to milk casein at each test temperature was derived. A relative value of the specific activity (relative activity (%)) at each test temperature when the maximum value among the obtained specific activities was taken as 100% was derived. The results showing the relationship between the relative activity and the temperature are shown in Fig. 4a.

As is apparent from Fig. 4a, the optimum temperature of the modified protein glutaminase A2V/T113A/N154D/N158H of the present invention (Example 1: solid line) for milk casein was 70°C, and was shifted to a temperature region higher than the optimum temperature (60°C) of the wild-type C. proteolyticum-derived protein glutaminase before modification (Comparative Example 1: dashed line) by about 10°C. Note that, when milk casein is used as a substrate, (unlike a low-molecular-weight substance such as Z-Gln-Gly and a plant protein such as soybean protein and pea protein), not only the protein glutaminase of Example 1 but also the protein glutaminase of Comparative Example 1 maintained a relatively high relative activity in a temperature region higher than the optimum temperature by 5°C.

### [4-2]

Similarly to the above [4-1], the milk casein is used as a substrate, and for the modified protein glutaminase A2V/S26P/T113A/N154D/N158H of the present invention (Example 2) and the wild-type C. proteolyticum-derived protein glutaminase before modification (Comparative Example 1, SEQ ID NO: 1), the specific activity at each test temperature (37°C, 50°C, 60°C, 65°C, 70°C, 75°C, or 80°C) was measured, and the results showing the relationship between the relative value of the specific activity (relative activity (%)) and the temperature are shown in Fig. 4b.

As is apparent from Fig. 4b, the optimum temperature of the modified protein glutaminase A2V/S26P/T113A/N154D/N158H of the present invention (Example 2: dashed-dotted line) for milk casein was 75°C, and was shifted to a temperature region higher than the optimum temperature (60°C) of the wild-type C. proteolyticum-derived protein glutaminase before modification (Comparative Example 1: dashed line) by about 15°C. The optimum temperature of the protein glutaminase of Example 2 for milk casein was shifted to a temperature region higher than the optimum temperature of the protein glutaminase of Example 1 shown in Fig. 4a by about 5°C.

### [Test Example 5]

In a 30 mL glass bottle, 1.2 g of oat flour (product name: Premium oat flour (manufactured by Slow Food Co., Ltd.), protein content: 13.1 wt%) was dispersed in 10 g of water to prepare a suspension (plant protein-containing liquid composition prepared using cereal crops). 10 U/g-starch α-amylase (α-amylase derived from Bacillus amyloliquefaciens, Amano Enzyme Inc.) and wild-type protein glutaminase (wild-type PG, Comparative Example 1, SEQ ID NO: 1) or the modified protein glutaminase of the present invention (PG-D4, Example 1, SEQ ID NO: 2) in amounts shown in Tables 1 and 2 were added, and a reaction was performed at 65°C or 70°C for 2 hours. That is, the treatment with α-amylase and the treatment with various PGs were simultaneously performed under common temperature conditions. After the reaction, the resulting product was heat-treated at 95°C for 5 minutes to inactivate the enzyme. Thereafter, centrifugation was performed under the conditions of 15,000 rpm and 10 minutes, and the supernatant was recovered. The free amount of oat protein in the recovered supernatant was quantified by the BCA method shown in the above (2), and the quantitative value was obtained as a protein concentration (mg/mL). For Examples 3-1 to 3-5 and Examples 4-1 to 4-4, the relative value of the protein concentration when the protein concentration in the corresponding comparative example (Comparative Examples 3-1 to 3-5 and Comparative Examples 5-1 to 5-4, respectively) was taken as 1 was derived as a solubility improvement index. The results are shown in Tables 1 and 2.

**[Table 1]**

| | Comparative Example | | | | | |
|---|---|---|---|---|---|---|
| | 2 | 3-1 | 3-2 | 3-3 | 3-4 | 3-5 |
| Wild-type PG (Comparative Example 1) | - | 0.26 | 0.77 | 2.79 | 8.26 | 13.5 |
| Added amount (µg/mL) | | | | | | |
| Treatment temperature (°C) | 65 | 65 | 65 | 65 | 65 | 65 |
| Protein concentration (mg/mL) | 1.72 | 1.53 | 1.71 | 2.95 | 3.81 | 3.84 |
| | | | | | | |

| | Comparative Example | **Example** | | | | |
|---|---|---|---|---|---|---|
| | 2 | **3-1** | **3-2** | **3-3** | **3-4** | **3-5** |
| **PG-D4 (Example 1)** | - | **0.26** | **0.77** | **2.79** | **8.26** | **13.5** |
| Added amount (µg/mL) | | | | | | |
| Treatment temperature (°C) | 65 | **65** | **65** | **65** | **65** | **65** |
| Protein concentration (mg/mL) | 1.72 | **1.89** | **2.21** | **3.91** | **4.12** | **4.14** |
| Solubility improvement index | | **1.24** | **1.29** | **1.33** | **1.08** | **1.08** |

**[Table 2]**

| | Comparative Example | | | | |
|---|---|---|---|---|---|
| | 4 | 5-1 | 5-2 | 5-3 | 5-4 |
| Wild-type PG (Comparative Example 1) | - | 0.26 | 0.77 | 2.79 | 13.5 |
| Added amount (µg/mL) | | | | | |
| Treatment temperature (°C) | 70 | 70 | 70 | 70 | 70 |
| Protein concentration (mg/mL) | 1.66 | 1.48 | 1.51 | 1.63 | 1.78 |
| | | | | | |

| | Comparative Example | **Example** | | | |
|---|---|---|---|---|---|
| | 4 | **4-1** | **4-2** | **4-3** | **4-4** |
| **PG-D4 (Example 1)** | - | **0.26** | **0.77** | **2.79** | **13.5** |
| Added amount (µg/mL) | | | | | |
| **Treatment** temperature (°C) | 70 | **70** | **70** | **70** | **70** |
| Protein concentration (mg/mL) | 1.66 | **1.6** | **2.17** | **3.32** | **3.61** |
| Solubility improvement index | | **1.08** | **1.44** | **2.04** | **2.03** |

Since the optimum temperature of the modified protein glutaminase of the present invention (Example 1, SEQ ID NO: 2) is higher than that of the wild-type protein glutaminase (Comparative Example 1, SEQ ID NO: 1), it was possible to more efficiently solubilize proteins by simultaneously treating the modified protein glutaminase under the common temperature condition with α-amylase having a higher optimum temperature than the wild-type protein glutaminase. Specifically, as shown in Examples 3-1 to 3-5 and Examples 4-1 to 4-4 using the modified protein glutaminase of the present invention, the solubility of protein could be improved as compared with Comparative Examples 3-1 to 3-5 and Comparative Examples 5-1 to 5-4 using the wild-type protein glutaminase, respectively. In Examples 3-1 to 3-5 treated at a temperature condition of 65°C, the solubility of protein could be improved up to 1.33 times as much as in the case of using the wild-type protein glutaminase, whereas in Examples 4-1 to 4-4 treated at a higher temperature condition of 70°C, the solubility of protein could be improved up to 2.04 times as much as in the case of using the wild-type protein glutaminase.

In the same manner as described above, a plant protein-containing liquid composition was prepared using oat flour, and the treatment with wild-type PG (Comparative Example 1) or PG-D4 (Example 1) was performed simultaneously with the treatment with α-amylase, and then the enzyme was inactivated. The obtained treated product (oat milk containing a modified oat protein material) was not subjected to centrifugation but stirred to foam. As a result, the foaming property and foam stability of the oat milk by the treatment with PG-D4 (Example 1) were improved as compared with the oat milk by the treatment with wild-type PG (Comparative Example 1). The treated product (oat milk containing a modified oat protein material) was not subjected to centrifugation, oil was added thereto, and the mixture was homogenized to emulsify the treated product. As a result, the emulsifiability and emulsion stability of the oat milk by the treatment with PG-D4 (Example 1) were improved as compared with the oat milk by the treatment with wild-type PG (Comparative Example 1).

### [Test Example 6]

Using various proteins shown in Table 3 or Z-Gln-Gly as a substrate (final concentration: 5 wt%), and using, as a protein glutaminase, the wild-type C. proteolyticum-derived protein glutaminase before modification (Comparative Example 1, SEQ ID NO: 1), the modified protein glutaminase A2V/T113A/N154D/N158H of the present invention (Example 1, SEQ ID NO: 2), or the modified protein glutaminase A2V/S26P/T113A/N154D/N158H of the present invention (Example 2), specific activity of the protein glutaminase was measured at a reaction temperature of 70°C in the same manner as in Test Examples 1 to 4. The specific activity at 70°C is shown in Table 4. Table 4 also shows the relative values of the specific activity of the protein glutaminases of Examples 1 and 2 when the specific activity of the protein glutaminase of Comparative Example 1 is taken as 1.

**[Table 3]**

| Product name | Protein type | Protein content | Manufacturer |
|---|---|---|---|
| SOYPRO | Soybean protein | 50 w/w% | J-OIL MILLS, INC. |
| NUTRALYS F85M | Pea protein | 85 w/w% | Roquette Japan K.K. |
| Fava protein | Fava bean protein | 80 w/w% | Ingredion |
| Chickpea protein | Chickpea protein | 84 w/w% | Organo Corporation |
| Mung bean protein | Mung bean protein | 80 w/w% | Ingredion |
| Lentil protein | Lentil bean protein | 50 w/w% | Organo Corporation |
| Almond protein | Almond protein | 85 w/w% | Bio Actives Japan Corporation |
| Oat protein | Oat protein | 75 w/w% | Organo Corporation |
| Wheat gluten | Wheat protein | 95 w/w% | Wako Pure Chemical Industries, Ltd. |
| Rice protein | Rice protein | 85 w/w% | Organo Corporation |
| Casein from bovine milk | Casein | 94 w/w% | Merck |
| MILEI protein | Whey protein | 90 w/w% | MORINAGA MILK INDUSTRY CO., LTD. |
| Egg albumin | Ovalbumin | 100 w/w% | Wako Pure Chemical Industries, Ltd. |

**[Table 4]**

| | Specific activity at 70°C (U/mg-protein) | | | | |
|---|---|---|---|---|---|
| | Comparative Example 1 | **Example 1** | | **Example 2** | |
| Z-Gln-Gly | 18.2 | **76.0** | **(4.2)** | **92.5** | **(5.1)** |
| Soybean protein | 13.9 | **83.9** | **(6.0)** | **108.4** | **(7.8)** |
| Pea protein | 5.2 | **34.3** | **(6.6)** | **46.8** | **(9.0)** |
| Fava bean protein | 10.2 | **71.6** | **(7.0)** | **87.7** | **(8.6)** |
| Chickpea protein | 8.1 | **52.2** | **(6.4)** | **71.1** | **(8.7)** |
| Mung bean protein | 13.3 | **80.3** | **(6.0)** | **94.9** | **(7.1)** |
| Lentil bean protein | 7.7 | **53.6** | **(7.0)** | **75.0** | **(9.8)** |
| Almond protein | 15.8 | **79.4** | **(5.0)** | **96.2** | **(6.1)** |
| Oat protein | 12.9 | **89.8** | **(6.9)** | **107.0** | **(8.3)** |
| Wheat protein | 33.1 | **95.6** | **(2.9)** | **102.4** | **(3.1)** |
| Rice protein | 2.4 | **9.5** | **(3.9)** | **14.2** | **(5.9)** |
| Casein | 35.6 | **118.3** | **(3.3)** | **133.0** | **(3.7)** |
| Whey protein | 10.7 | **59.8** | **(5.6)** | **83.3** | **(7.8)** |
| Ovalbumin | 6.0 | **43.0** | **(7.1)** | **52.6** | **(8.7)** |

The numerical value in parentheses is a relative value when the specific activity in Comparative Example 1 is taken as 1.

It was confirmed that even when any protein was used as a substrate, the optimum temperatures of the protein glutaminase of Example 1 and the protein glutaminase of Example 2 were higher than the optimum temperature of the protein glutaminase of Comparative Example 1. As shown in Table 4, the specific activity at 70°C was remarkably improved in the protein glutaminase of Example 1 and the protein glutaminase of Example 2 as compared with the protein glutaminase of Comparative Example 1. The specific activity at 70°C was improved in the protein glutaminase of Example 2 as compared with the protein glutaminase of Example 1.

## Claims

1. A modified protein glutaminase comprising a polypeptide shown in any one of (I) to (III) below:
(I) a polypeptide consisting of an amino acid sequence in which, in the amino acid sequence set forth in SEQ ID NO: 1, at least one substitution has been introduced from among a substitution of the amino acid residue at position 2 to a valine residue, a substitution of the amino acid residue at position 113 to an alanine residue, a substitution of the amino acid residue at position 154 to an aspartic acid residue, and a substitution of the amino acid residue at position 158 to a histidine residue;
(II) a polypeptide in which one or several amino acid residues other than the substituted amino acid residues are substituted, added, inserted, or deleted in the amino acid sequence in which the substitution has been introduced, and an optimum temperature is higher than an optimum temperature of a polypeptide consisting of the amino acid sequence set forth in SEQ ID NO: 1, and
(III) a polypeptide in which a sequence identity of regions excluding the substituted amino acid residues is 70% or more in the amino acid sequence in which the substitution has been introduced, and an optimum temperature is higher than an optimum temperature of a polypeptide consisting of the amino acid sequence set forth in SEQ ID NO: 1.

2. A DNA encoding the modified protein glutaminase according to claim 1.

3. An expression cassette or recombinant vector comprising the DNA according to claim 2.

4. A transformant obtained by transforming a host using the expression cassette or recombinant vector according to claim 3.

5. A method for producing a modified protein glutaminase, the method comprising a step of culturing the transformant according to claim 4.

6. An enzyme preparation comprising the modified protein glutaminase according to claim 1.

7. A modifier for a protein material, comprising the modified protein glutaminase according to claim 1.

8. A method for producing a modified protein material, the production method comprising an enzyme treatment step of allowing the modified protein glutaminase according to claim 1 to act on a protein material.

9. The production method according to claim 8, wherein the protein material is a plant protein material.

10. The production method according to claim 8, wherein the protein material is provided in the form of a plant protein-containing liquid composition prepared using cereal crops, and
in the enzyme treatment step, the plant protein-containing liquid composition is simultaneously treated with a modified protein glutaminase and an amylase.
